# EUROPEAN PATENT APPLICATION

(11) **EP 4 682 546 A1**
(43) Date of publication of application: **21.01.2026**
(21) Application number: 23927717.1
(22) Date of filing: 16.11.2023
(51) Int. Cl.: G01N 35/10

(54) **LIQUID HANDLING DEVICE**

(30) Priority: 15.03.2023 KR 20230033671
(71) Applicant: S-biomedics Co., Ltd., Seoul 04797 (KR)
(72) Inventor: KIM, Jong Wan, Seoul 02714 (KR); KWON, Ji Hye, Seoul 03403 (KR); CHO, Hyun Ju, Goyang-si, Gyeonggi-do 10535 (KR)
(74) Representative: Zacco Sweden AB
(86) International application number: PCT/KR2023/018408
(87) International publication number: WO 2024/190997

(57) **Abstract**

The present invention relates to a liquid handling device, and more particularly, to a liquid handling device that automates liquid handling for a cell culture process, thereby improving precision and reproducibility.

## Description

### Technical Field

One or more embodiments relate to a liquid handling device.

### Background Art

In the conventional industry, 'liquid handling' is a technology of handling liquid substances to perform biochemical, chemical, and biological tests. Liquid handling is generally defined as a work of bringing one sample into contact with another, but may sometimes include quantifying at least one of samples being used.

Furthermore, liquid handling may be strictly limited only to a work on liquid substances, but hereinafter it is intended to include general operations on solids, liquids, or gases, or mixtures thereof (e.g., heterogeneous samples containing solids and liquids, such as cell culture media).

These days, liquid handling is carried out manually by individuals or by various types of automated devices. However, in conventional laboratory environments, liquid handling was mainly carried out manually by individuals.

Manual liquid handling is carried out by a worker using volume-adjustable pipettes that may transfer liquid from one container to another in a predefined, known quantity.

However, manual liquid handling has disadvantages in that precision and reproducibility depend on the skill of the operator, and liquid samples may evaporate during operation or may be partially lost due to being stuck to pipettes.

In general, large-scale, repetitive liquid handling tasks are essential in bio-experiments using cells or in the production process of bioproducts. In case that such task is performed by manual liquid handling, high training costs and investments in facilities are required to ensure workforce for mass cell production, and even in case that skilled workers are available, productivity may be reduced due to differences in quality or yield per production lot, which may arise from factors, such as workers and work environments.

### Disclosure of Invention

### Technical Problem

One or more embodiments provide a liquid handling device that enhances precision and reproducibility of a cell culture process and improves productivity and quality of cells, by automating liquid handling for the cell culture process.

### Solution to Problem

According to one or more embodiments, a liquid handling device includes a well plate having a plurality of culture wells, a work unit configured to move the well plate along a preset path, a dispenser unit configured to dispense or aspirate at least one of a coating solution and a culture medium into or from the well plate using pipette tips, a gripper unit configured to grip the well plate and move the well plate along a preset path, and a reservoir unit storing the at least one of the coating solution and the culture medium to supply the same to the dispenser unit.

### Advantageous Effects of Invention

According to one or more embodiments, a liquid handling device may enhance workers' convenience and improve the precision and reproducibility of liquid handling by automating liquid handling for a coating solution and culture medium for a cell culture system.

According to one or more embodiments, a liquid handling device may enable mass production of cells by continuously and repeatedly processing liquid handling and cell seeding, and may evenly maintain the quality of cultured cells.

According to one or more embodiments, a liquid handling device may dispense a coating solution or culture medium in a plurality of stages, through a splitting operation during liquid handling. Accordingly, efficient liquid handling may be allowed and various types of well plates may be applied to the device.

Of course, the scope of the present disclosure is not limited by these effects.

### Brief Description of Drawings

FIG. 1 is a block diagram of a cell culture system using a liquid handling device according to an embodiment.
FIG. 2 is a perspective view of a liquid handling device according to an embodiment.
FIG. 3 is an enlarged view of a region A of FIG. 2.
FIG. 4 is an enlarged view of a region B of FIG. 2.
FIG. 5 is a view of a base of FIG. 2, viewed from above.
FIG. 6 is a view of a state in which a gripper unit of FIG. 2 grips a well plate.
FIG. 7 is a view of a state in which the gripper unit of FIG. 2 places the well plate on a worktable.
FIGS. 8 and 9 are views of a method of dispensing a solution into a well plate by a dispenser unit.
FIG. 10 is a view of a process of coating a well plate using the dispenser unit of FIG. 2.
FIG. 11 is a view of a process of seeding and culturing cells using the dispenser unit of FIG. 2.
FIG. 12 is a flowchart of a cell culturing method using a liquid handling device according to an embodiment.

### Best mode for Carrying out the Invention

According to one or more embodiments, a liquid handling device includes a well plate having a plurality of culture wells, a work unit configured to move the well plate along a preset path, a dispenser unit configured to dispense or aspirate at least one of a coating solution and a culture medium into or from the well plate using pipette tips, a gripper unit configured to grip the well plate and move the well plate along a preset path, and a reservoir unit storing the at least one of the coating solution and the culture medium therein to supply the same to the dispenser unit.

The dispenser unit may dispense the coating solution stored in the reservoir unit into the well plate and dispense the culture medium into the well plate coated with the coating solution.

The dispenser unit may dispense the culture medium after aspirating the dispensed coating solution.

The dispenser unit may mix the culture medium stored in the reservoir unit with cells and then dispense the culture medium mixed with the cells into the well plate.

The well plate may have a coating surface which is coated with the coating solution in each of the culture wells, with the cells in the culture media aggregating into a spheroidal shape on the coating surface.

The working unit may include a loading part on which the well plate is loaded, a worktable on which the well plate is placed, such that the coating solution or the culture medium is dispensed into the well plate, and an unloading part by which the dispensed well plate is unloaded.

### Mode for the Invention

As the disclosure allows for various changes and numerous embodiments, particular embodiments will be illustrated in the drawings and described in detail in the written description. Effects and features of the disclosure and methods of achieving the same will be apparent with reference to embodiments and drawings described below in detail. The disclosure may, however, be embodied in many different forms and should not be construed as being limited to the embodiments set forth herein.

Hereinafter, a description will be given in detail of embodiments disclosed herein, with reference to the accompanying drawings. For description with reference to the drawings, the same or equivalent components may be given the same reference numerals, and a redundant description thereof will be omitted.

To clearly explain the disclosure in the drawings, parts that are not related to the description are omitted, and similar parts are given similar reference numerals throughout the disclosure.

In the following embodiments, terms, such as first and second, are used not in a limiting sense but for the purpose of distinguishing one component from another component.

The singular forms as used herein are intended to include the plural forms as well unless the context clearly indicates otherwise.

In the embodiments disclosed below, terms such as "include" or "has" should be understood that they are intended to indicate an existence of features or components, disclosed in this specification, and also it is not excluded in advance that one or more features or components are likewise utilized.

In the following embodiments, it will be understood that, when a portion such as unit, region, or element is referred to as being "on" another portion, this may include not only a case where the portion is directly on the other portion, but also a case where intervening films, regions, or elements may be present therebetween.

In the following embodiments, the terms, such as "connected" or "coupled" do not necessarily mean "two members being directly and/or fixedly connected or coupled," unless otherwise specified within the context, and do not exclude the intervention of other members between the two members.

Sizes of elements in the drawings may be exaggerated for convenience of explanation. For example, a size and a thickness of each component illustrated in the drawings are illustrative for convenience of description, and the embodiment below is not limited to the size and the thickness of the component illustrated.

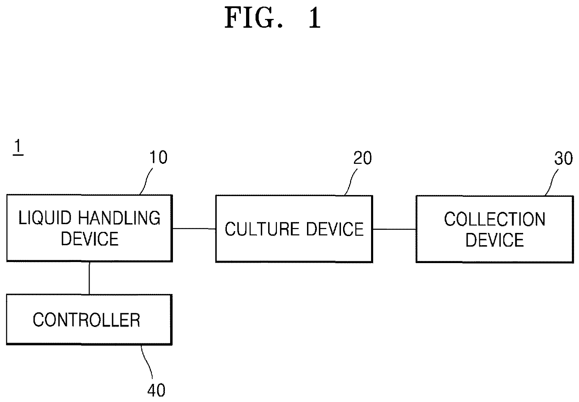

FIG. 1 is a block diagram of a cell culture system using a liquid handling device according to an embodiment.

Referring to FIG. 1, a cell culture system 1 may include a liquid handling device 10, a culture device 20, a collection device 30, and a controller 40.

The liquid handling device 10 may move a well plate along a preset path by operation of the controller 40. The well plate may generally refer to a culture dish widely used in bio-experiments and may have a plurality of culture wells. Cells may be cultured by dispensing the cells and a culture medium into the plurality of culture wells.

The liquid handling device 10 may quantitatively transfer a liquid material to the well plate by the operation of the controller 40. The liquid material may be a solution for cell culture.

The liquid handling device 10 may expel the liquid material into the well plate, and may aspirate the liquid material contained in the well plate. The liquid handling device 10 may mix the solution by repeating the aspiration and expulsion of the liquid material.

The liquid handling device 10 may dispense, aspirate, or mix the liquid material. Hereinafter, the dispensing, aspiration, or mixing operation for the liquid material will be commonly defined as 'liquid handling.'

According to an embodiment, the liquid handling device 10 may evenly mix a solution that cells are added in a culture medium by repeating the aspiration and expulsion.

The liquid handling device 10 may seed the cells by aspirating the culture medium mixed with the cells and dispensing the aspirated culture medium into the well plate.

The culture device 20 may be a device that cultures the cells by receiving the well plate in which the cells have been seeded.

The culture device 20 may be adjustable for temperature, humidity, and other factors to create suitable environments for cell cultures. For example, the culture device 20 may be configured as a typical incubator.

The collection device 30 may receive the well plate in which the cell culture has been completed and collect cultured cell clusters. The collection device 30 may separate and transport the cell clusters from the well plate.

In some embodiments, the collection device 30 may be arranged as a separate device, but may be implemented as the liquid handling device 10, so that the liquid handling device 10 may collect the cell clusters.

The liquid handling device 10 may receive the well plate in which the culture has been completed, aspirate the cell clusters from the well plate, expel the cell clusters into a collection container, and collect the cell clusters.

The cell culture system 1 may seed and culture cells in as many well plates as possible through repeated operations of the liquid handling device 10, the culture device 20, the collection device 30, and the controller 40. Accordingly, the cell culture system 1 may enable mass production of cells and produce the cells with more uniform quality.

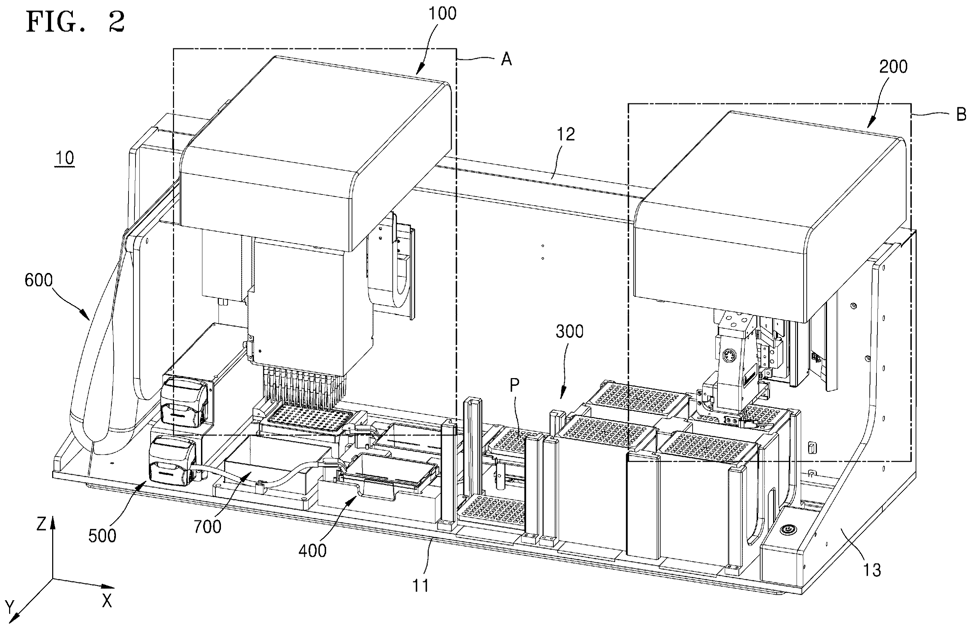

FIG. 2 is a perspective view of a liquid handling device according to an embodiment. Hereinafter, to explain the shape of the liquid handling device 10 or the movement of components, a left-right direction is defined as an X-axis direction, a back-and-forth direction is defined as a Y-axis direction, and an up-and-down direction is defined as a Z-axis direction based on the liquid handling device 10.

Referring to FIG. 2, the liquid handling device 10 may have a housing that includes a base 11, a rear housing 12, and side housings 13.

The base 11 may be a lower plate on which components of the liquid handling device 10 are arranged.

The base 11 may be formed in a plate shape that has a width in the X-axis direction and a length in the Y-axis direction.

On the base 11, a work unit 300, a reservoir unit 400, a pump unit 500, and a waste disposal unit 700 may be arranged.

The rear housing 12 may form a rear surface portion of the liquid handling device 10.

The rear housing 12 may be connected vertically to the base 11 to have a width in the X-axis direction and a height in the Z-axis direction.

The rear housing 12 may have a dispenser unit 100 and a gripper unit 200 mounted thereto. The dispenser unit 100 and the gripper unit 200 may be movably installed with respect to the rear housing 12.

According to an embodiment, the dispenser unit 100 and the gripper unit 200 may be movable in the X-axis direction in the liquid handling device 10 along the width of the rear housing 12.

The dispenser unit 100 and the gripper unit 200 may be positioned apart from the base 11 in the Z-axis direction by the height of the rear housing 12.

The dispenser unit 100 and the gripper unit 200 may each include an actuator arranged in a partial region to be movable in the X-axis, Y-axis, and Z-axis directions.

The side housings 13 may be coupled to the base 11 and the rear housing 12 to form side surface portions of the liquid handling device 10.

The side housings 13 may assist vertical coupling between the base 11 and the rear housing 12, so that components arranged on the base 11 and the rear housing 12 may be stably arranged and operated.

The liquid handling device 10 may include a well plate P, a dispenser unit 100, a gripper unit 200, a work unit 300, a reservoir unit 400, a pump unit 500, a sterilization bag 600, and a waste disposal unit 700.

The well plate P may be a culture dish that is generally widely used in bio-experiments. The well plate P may include a plurality of culture wells. In the plurality of culture wells, a liquid material transported by the dispenser unit 100 may be dispensed.

The liquid material may be a solution for culturing cells to produce cell clusters. In some embodiments, the solution may include a coating solution, a buffer, or a culture medium.

The dispenser unit 100 may be arranged on one side of the rear housing 12.

The dispenser unit 100 may be installed on the rear housing 12 to be movable along a preset path on the X-axis, Y-axis, and Z-axis.

The dispenser unit 100 may quantitatively transport the solution.

The dispenser unit 100 may be configured to dispense or aspirate the solution in a preset volume. The dispenser unit 100 may be configured to mix the solution by repeating aspiration and expulsion.

The dispenser unit 100 may perform liquid handling for the solution in the liquid handling device 10. The dispenser unit 100 may dispense the solution into the culture wells of the well plate P or aspirate the solution from the culture wells of the well plate P.

The dispenser unit 100 may have typical mechanical pipettes or similar structures embedded therein. In general, mechanical pipettes may be used by attaching pipette tips. The pipette tip may be a disposable consumable that performs liquid handling by coming into contact with a sample. Therefore, the sample may not be introduced into the pipette, and contamination for the solution may be prevented. The pipette tips may be detachably mounted to the dispenser unit 100.

The gripper unit 200 may be arranged on another side of the rear housing 12 and may be positioned on an opposite side to the dispenser unit 100.

The gripper unit 200 may be installed on the rear housing 12 to be movable along a preset path on the X-axis, Y-axis, and Z-axis.

The gripper unit 200 may pick up the well plate P, move the well plate P along the preset path, and arrange the well plate P on an appropriate position. At this time, the dispenser unit 100 may perform liquid handling with respect to the arranged well plate P.

The work unit 300 may be arranged on the base 11 to be adjacent to the gripper unit 200. The work unit 300 may be located below the gripper unit 300.

The work unit 300 may designate a position where the well plate P is loaded or moved to be arranged.

The work unit 300 may be arranged with a portion adjacent to the dispenser unit 100. At this time, the dispenser unit 100 may perform liquid handling for the well plate P arranged in the work unit 300.

The reservoir unit 400 may be arranged on the base 11 to be adjacent to the dispenser unit 100. The reservoir unit 400 may be arranged between the dispenser unit 100 and the gripper unit 200.

The reservoir unit 400 may form a space in which the solution is stored. The reservoir unit 400 may have a tank shape.

In case that the solution is stored in the reservoir unit 400, the dispenser unit 100 may perform liquid handling for the stored solution.

For example, the dispenser unit 100 may aspirate the solution from the reservoir unit 400 and move the aspirated solution to the well plate P arranged in the work unit 300.

The dispenser unit 100 may uniformly mix the solution by repeatedly aspirating and expelling the solution stored in the reservoir unit 400.

The pump unit 500 may be arranged on the base 11. The pump unit 500 may be arranged adjacent to the reservoir unit 400, but does not have to be arranged directly next to the reservoir unit 400.

The pump unit 500 may be a typical tubing pump, and may generate pressure to fill the solution in the reservoir unit 400.

The pump unit 500 may replenish the solution by an amount that the solution stored in the reservoir unit 400 decreases.

The sterilization bag 600 may be arranged next to the pump unit 500. The sterilization bag 600 may be detachably installed on one side of the base 11, the rear housing 12, or the side housing 13.

The liquid handling device 10 may further include a tube connecting the sterilization bag 600 to the reservoir unit 400. The pump unit 500 may move the solution stored in the sterilization bag 600 to the reservoir unit 400 through the tube.

The sterilization bag 600 may be a bag for storing the solution in a sterile state, and the solution may be one selected from among a coating solution, a buffer, or a culture medium.

The sterilization bag 600 may be arranged as a plurality of sterilization bags, and may be replaced as needed. For example, a sterilization bag (not shown) in which a coating solution is stored may be used by being installed in the liquid handling device 10, and then may be replaced with another sterilization bag (not shown) in which a buffer is stored.

The waste disposal unit 700 may be arranged on the base 11 to be adjacent to the dispenser unit 100. The waste disposal unit 700 may be arranged below the dispenser unit 100, and the waste disposal unit 700 may be arranged between the reservoir unit 400 and the pump unit 500.

The waste disposal unit 700 may form a space in which a waste solution or used pipette tips are discarded. The waste disposal unit 700 may have a tank shape.

The waste disposal unit 700 may be connected to a separate drain pump (not shown) to discharge the waste solution.

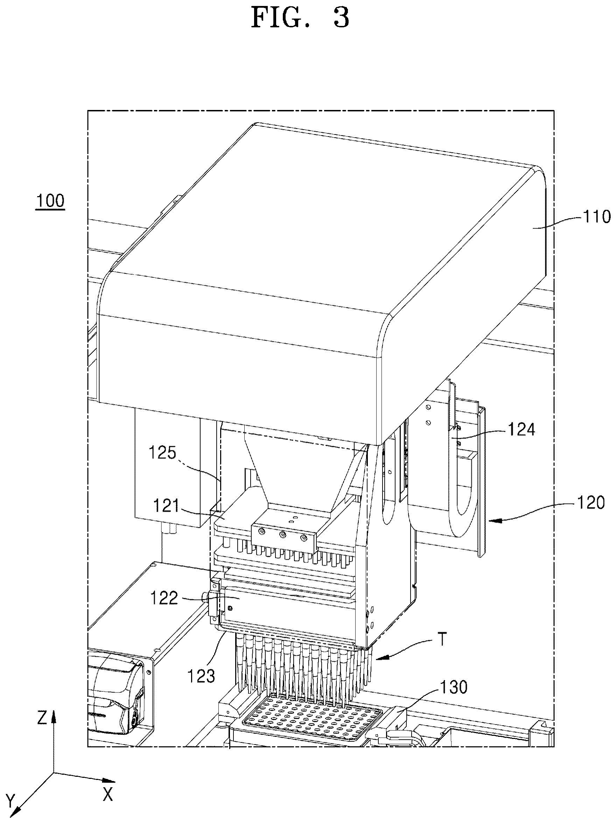

FIG. 3 is an enlarged view of a region A of FIG. 2, showing a detailed structure of the dispenser unit 100.

Referring to FIG. 3, the dispenser unit 100 may include a first driving part 110, a pipette part 120, and a pipette tip loading part 130.

The first driving part 110 may be the uppermost part of the dispenser unit 100 and may be movably connected to the rear housing 12.

The first driving part 110 may have a width in the X-axis direction and a length in the Y-axis direction, and may be installed parallel to the base 11.

The first driving part 110 may generate a driving force to move the dispenser unit 100. An X-axis actuator may be mounted at a position where the first driving part 110 and the rear housing 12 are connected, and a Y-axis actuator may be mounted inside the first driving part 110. Accordingly, the dispenser unit 100 may move in the X-axis direction or the Y-axis direction.

The pipette part 120 may be located on a lower surface of the first driving part 110 and may be installed to extend toward the base 11.

The pipette part 120 may include a plunger 121, a cylinder 122, a pipette tip coupling portion 123, a pipette actuator 124, and a cover 125.

A portion of the plunger 121 may be inserted into the cylinder 122.

The cylinder 122 may form a space into which the portion of the plunger 121 is inserted, and may include a through hole formed in a downward direction.

The plunger 121 and the cylinder 122 may be coupled to each other to operate on the same principle as a typical mechanical pipette, and air may move through the through hole.

The plunger 121 and the cylinder 122 may be arranged in a matrix shape in which a plurality of pipettes are arranged vertically and horizontally.

In case that the plunger 121 moves upward, the interior of the cylinder 122 may be depressurized, and an aspiration operation may be performed through the through hole. In another example, in case that the plunger 121 moves downward, the interior of the cylinder 122 may be pressurized, and an expulsion operation may be performed through the through hole.

By the aspiration or expulsion operation of the plunger 121 and the cylinder 122, the liquid handling device 10 may perform liquid handling for the solution.

The pipette tip coupling portion 123 may be arranged on an end of the pipette part 120.

The pipette tip coupling portion 123 may extend from the through hole located on a lower surface of the cylinder 122.

The pipette tip coupling portion 123 may be detachably coupled with pipette tips T.

The pipette tip coupling portion 123 may be in direct contact with the solution through the pipette tips T during liquid handling.

The solution may be introduced into or discharged out of the pipette tips T according to the operation of the plunger 121 and the cylinder 122.

For example, in case that the pipette tips T are coupled to the pipette tip coupling portion 123, the pipette tips T may aspirate or expel the solution by the aspiration or expulsion operation of the plunger 121 and the cylinder 122.

The pipette actuator 124 may be located on a rear surface portion or side surface portion of the pipette part 120.

The pipette actuator 124 may be configured as a Z-axis actuator to move the pipette part 120 in the Z-axis direction.

Accordingly, the pipette part 120 may be moved along the Z-axis by the pipette actuator 124 to couple the pipette tips T to the pipette tip coupling portion 123.

In some embodiments, the pipette part 120 may be moved along the X-axis and Y-axis by the first driving part 110 to be located above the work unit 300, the reservoir unit 400, or the waste disposal unit 700.

The pipette part 120 may be moved along the Z-axis by the pipette actuator 124, and the plunger 121 and the cylinder 122 may be in contact with the solution or perform liquid handling for the solution.

The cover 125 may cover the plunger 121 and the cylinder 122 to form the appearance of the pipette part 120. In this way, even in case that the plunger 121 moves up and down while inserted in the cylinder 122, the cover 125 may prevent an introduction of foreign substances or work errors.

The pipette tip loading part 130 may be arranged on the base 11 to oppose the dispenser unit 100 in the up-and-down direction.

The pipette tips T prior to use may be arranged on the pipette tip loading part 130. The pipette tips T arranged on the pipette tip loading part 130 may be coupled to the pipette tip coupling portion 123.

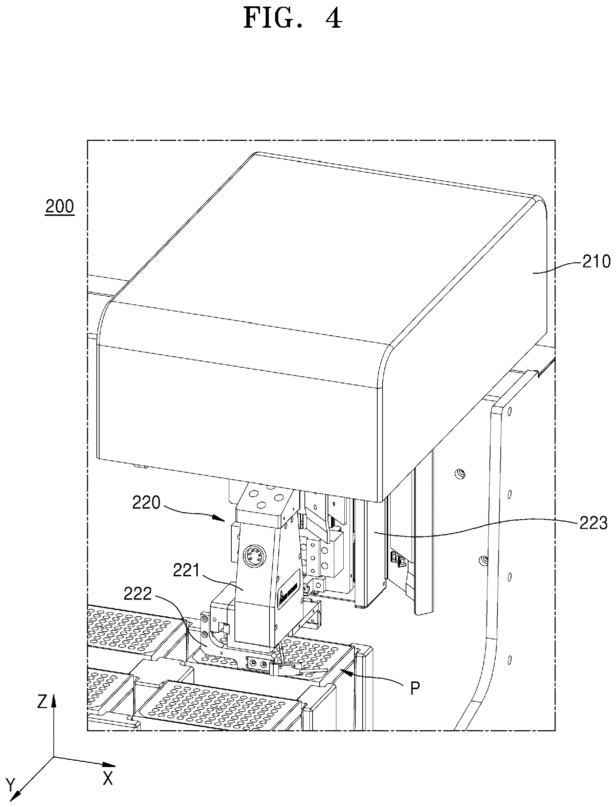

FIG. 4 is an enlarged view of a region B of FIG. 2, showing a detailed structure of the gripper unit 200.

Referring to FIG. 4, the gripper unit 200 may include a second driving part 210 and a grip part 220.

The second driving part 210 may be the uppermost part of the gripper unit 200 and may be movably connected to the rear housing 12.

The second driving part 210 may have a width in the X-axis direction and a length in the Y-axis direction, and may be installed parallel to the base 11.

The second driving part 210 may generate a driving force to move the gripper unit 200. An X-axis actuator may be mounted at a position where the second driving part 210 and the rear housing 12 are connected, and a Y-axis actuator may be mounted inside the second driving part 210. Accordingly, the gripper unit 200 may move in the X-axis direction or the Y-axis direction.

The grip part 220 may be located on a lower surface of the second driving part 210 and may be installed to extend toward the base 11.

The grip part 220 may include an extension arm 221, a gripper 222, and an arm actuator 223.

The extension arm 221 may have a structure that extends downward, and may be configured to approach the well plate P arranged in the work unit 300.

The gripper 222 may be arranged on an end of the extension arm 221.

The gripper 222 may perform a gripping operation for the well plate P and may be appropriately located to lift the well plate P.

The arm actuator 223 may be located on a rear surface portion or side surface portion of the extension arm 221.

The arm actuator 223 may be configured as a Z-axis actuator to move the extension arm 221 and the gripper 222 in the Z-axis direction.

Accordingly, the extension arm 221 and the gripper 222 may be moved along the Z-axis by the arm actuator 223, and may approach the well plate P arranged in the work unit 300 to perform the gripping operation or place the well plate P on a specific position.

The extension arm 221 and the gripper 222 may be moved along the X-axis and the Y-axis by the second driving part 210, so that the well plate P gripped by the gripper 222 may be moved along a preset path.

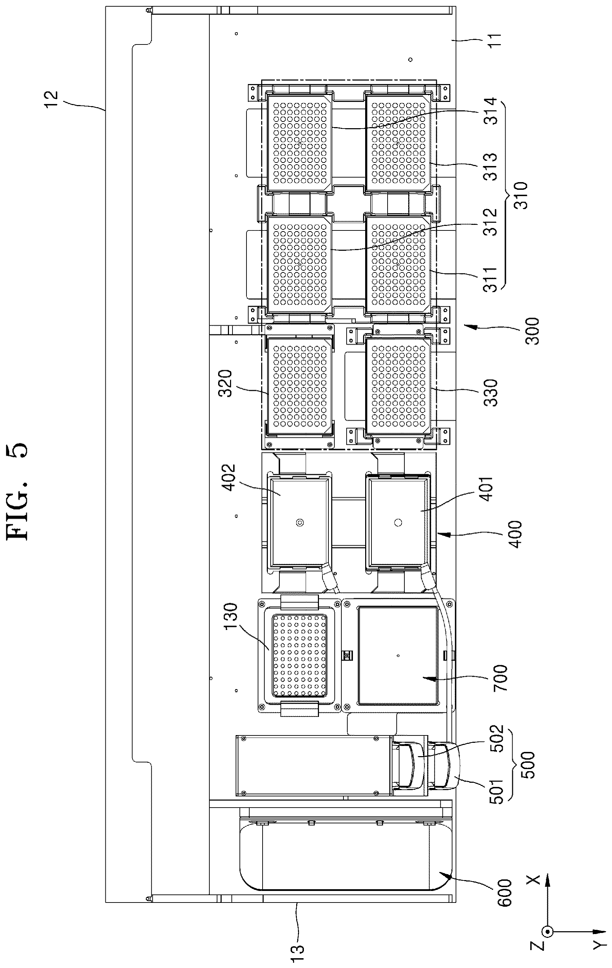

FIG. 5 is a view of a base of FIG. 2, viewed from above, showing an arrangement structure of components on the base 11. Hereinafter, those components will be listed and described in the order from left to right of the base 11 shown in the drawing.

On the base 11 may be arranged the sterilization bag 600, the pump unit 500, the pipette tip loading part 130, the reservoir unit 400, and the work unit 300.

The sterilization bag 600 may store a sterile solution therein, and the solution may be a coating solution or a culture medium.

A tube may be connected to the sterilization bag 600, and an opposite side of the tube may be connected to the reservoir unit 400. The pump unit 500 may move the solution stored in the sterilization bag to the reservoir unit 400 through the tube.

The pump unit 500 may include a plurality of tubing pumps. For example, the pump unit 500 may include a first pump 501 and a second pump 502.

The pipette tip loading part 130 may be arranged between the pump unit 500 and the reservoir unit 400, and may oppose the pipette part 120 before operation or in a standby state.

For example, in case that the dispenser unit 100 is before operation or in a standby state, the pipette part 120 may be stopped above the pipette tip loading part 130. This may be defined as a standby position of the dispenser unit 100.

The waste disposal unit 700 may be arranged in front of the pipette tip loading part 130 and located between the pump unit 500 and the reservoir unit 400. Accordingly, the dispenser unit 100 may easily discard a waste solution or used pipette tips T, which are generated during the liquid handling process, into the waste disposal unit 700.

The reservoir unit 400 may be arranged as a plurality of reservoir units, and may include a first reservoir 401 and a second reservoir 402. The first reservoir 401 and the second reservoir 402 may store the same solution or may store different types of solutions, respectively, for use.

As described above in FIG. 2, the pump unit 500 may replenish the solution by an amount that the solution stored in the reservoir unit 400 is decreased by the aspiration operation of the pipette part 120.

According to an embodiment, the liquid handling device 10 may be configured such that the first pump 501 transfers a first solution to the first reservoir 401 and the second pump 502 transfers a second solution to the second reservoir 402. For example, the first solution may be a coating solution, and the second solution may be a culture medium. However, the first solution and the second solution are not limited to these, and may be applied and used as two types of solutions which are used continuously.

The work unit 300 may be arranged adjacent to the reservoir unit 400. The work unit 300 may be located on a right side of the reservoir unit 400. Accordingly, the dispenser unit 100 that transfers the solution from the reservoir unit 400 to the work unit 300 may be efficiently operated.

The work unit 300 may include a loading part 310, a worktable 320, and an unloading part 330.

A plurality of well plates P which are unused may be loaded on the loading part 310.

According to an embodiment, the loading part 310 may have four sections. The loading part 310 may include a first section 311, a second section 312, a third section 313, and a fourth section 314. The plurality of well plates P may be loaded on each of the first section 311, the second section 312, the third section 313, and the fourth section 314.

The loading part 310 may be arranged on an upper right side of the base 11 and may oppose the grip part 220 which is before operation or in the standby state.

For example, in case that the gripper unit 200 is before operation or in the standby state, the grip part 220 may be stopped above the loading part 310. This may be defined as a standby position of the gripper unit 200. In some embodiments, the standby position of the gripper unit 200 may be the fourth section 314.

The worktable 320 may be a space where the well plate P is arranged by the gripper unit 200 and liquid handling is performed by the dispenser unit 100.

The worktable 320 may be arranged between the reservoir unit 400 and the loading part 310. Accordingly, the worktable 320 may facilitate approach and operation of the dispenser unit 100 and the gripper unit 200.

The well plate P for which the liquid handling has been completed may be unloaded by being moved to the unloading part 330.

The unloading part 330 may be positioned in front of the worktable 320, so that a worker may easily take the well plate P out of the liquid handling device 10.

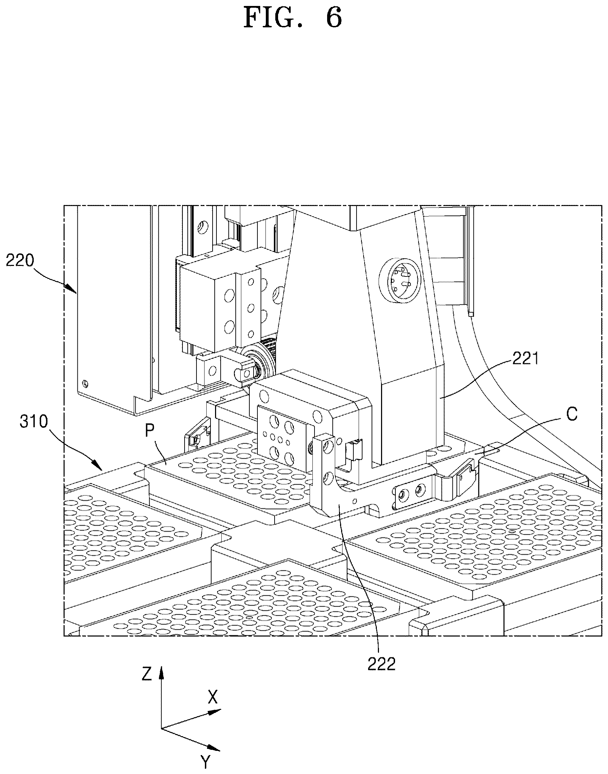

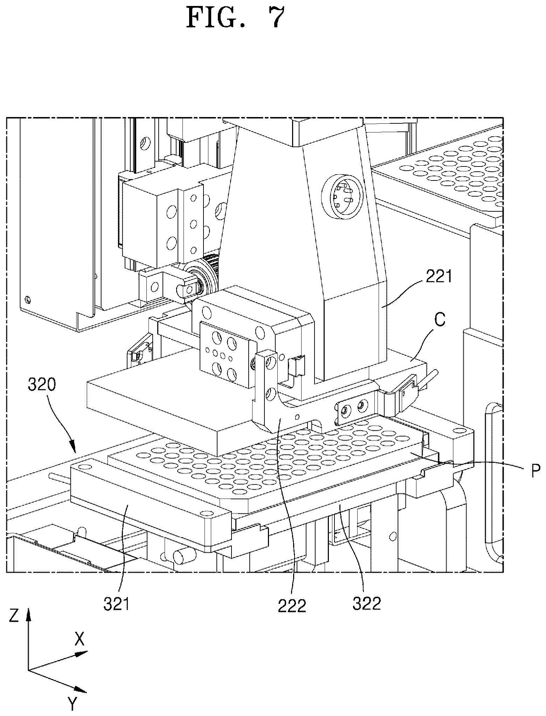

FIG. 6 is a view of a state in which a gripper unit of FIG. 2 grips a well plate, and FIG. 7 is a view of a state in which the gripper unit of FIG. 2 places the well plate on a worktable.

Referring to FIG. 6, as the extension arm 221 of the grip part 220 moves downward, the gripper 222 may come into contact with the well plate P placed on the top of the loading part 310.

At this time, the plurality of well plates P loaded on the loading part 310 may all be covered with a plate cover C, and unless otherwise stated, the well plates P may be considered to be moved together with the plate cover C.

According to an embodiment, the gripper 222 may have a width that is adjustable in the Y-axis direction. The gripper 222 may lift the well plate P by spreading along the width of the well plate P in the Y-axis direction.

As described above in FIGS. 2 and 4, the gripper unit 200 may be moved along a preset path on the X-axis or Y-axis by the second driving part 210, and the grip part 220 may be moved up and down along the Z-axis by the arm actuator 223.

Accordingly, after lifting the well plate P, the grip part 220 may move the well plate P along the preset path by the second driving part 210 and the arm actuator 223.

Referring to FIG. 7, the extension arm 221 may move along a preset path to place the well plate P gripped by the gripper unit 200 on the worktable 320.

The worktable 320 may support the well plate P, and the gripper 222 may accurately arrange the well plate P on the worktable 320.

Optionally, the worktable 320 may detachably fix the well plate P, so that the well plate P may be placed on an accurate position.

The worktable 320 may include first supporters 321 that support opposite side surfaces of the well plate P, and a second supporter 322 that supports a lower surface of the well plate P. The worktable 320 may fix and place the well plate P by adjusting a gap between the first supporters 321 according to the width of the well plate P in the X-axis direction.

In some embodiments, the well plate P placed on the worktable 320 has been previously transported while covered with the plate cover C, but may be separated from the plate cover C by the grip part 220.

According to an embodiment, after the well plate P is arranged on the worktable 320, the gripper 222 may grip and move only the plate cover C.

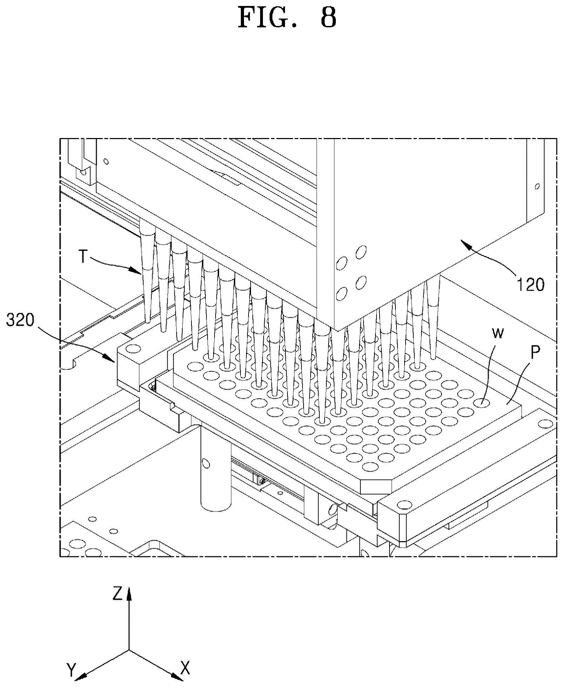

FIG. 8 is a view of a state in which the dispenser unit 100 dispenses a solution in the well plate P according to an embodiment.

Referring to FIG. 8, the number of pipette tips T mounted on the pipette part 120 may be the same as the number of culture wells w of the well plate P. A gap between the pipette tips T may be the same as a gap between the culture wells w.

As described above in FIGS. 2 and 3, the dispenser unit 100 may move along a preset path on the X-axis or Y-axis by the first driving part 110, and the pipette part 120 may be moved up and down along the Z-axis by the pipette actuator 124.

Accordingly, the pipette part 120 may be moved along a preset path by the first driving part 110 to be positioned above the well plate P arranged on the worktable 320. The pipette part 120 may adjust a distance between the pipette tips T and the well plate P by the pipette actuator 124.

According to an embodiment, the pipette part 120 may perform an expulsion operation to expel the solution contained in the pipette tips T into the plurality of culture wells w.

The pipette part 120 may perform an aspiration operation to aspirate the solution contained in the plurality of culture wells w.

The pipette part 120 may mix the solution contained in the plurality of culture wells w by performing a mixing operation of repeatedly performing the aspiration and expulsion.

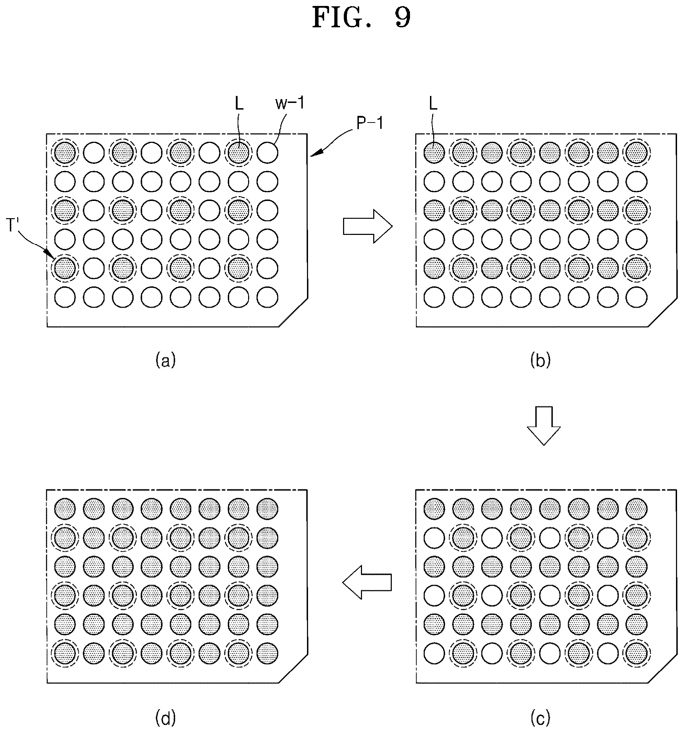

FIG. 9 is a view of a state in which the dispenser unit 100 dispenses a solution L into a well plate P-1 according to another embodiment.

For convenience of explanation, the dispenser unit 100 and the worktable 320 are omitted in FIG. 9, and only the well plate P-1 is shown as viewed from above. However, positions T' of the pipette tips for each dispensing step are indicated by dotted lines on the well plate P-1.

Referring to FIG. 9, the number of culture wells w-1 of the well plate P-1 may be greater than the number of pipette tips T mounted on the pipette part 120. A gap between the pipette tips T may be wider than a gap between the culture wells w-1.

The pipette part 120 may dispense the solution by a certain volume in a plurality of stages.

For example, the pipette part 120 may dispense the solution L contained in the corresponding pipette tip T a plurality of times through a splitting operation.

According to an embodiment, the pipette part 120 may dispense the solution L contained in the pipette tip T in four times.

For example, the pipette part 120 may aspirate 200 *µ*ℓ of solution L into the pipette tip T by an aspiration operation. The pipette part 120 may move to a position above the well plate P-1 placed on a worktable (not shown).

The pipette part 120 may dispense 50 *µ*ℓ of solution L into each culture well w-1 in each of four stages while changing the position T' of the pipette tip in a clockwise direction, as shown in (a) to (d) of FIG. 9.

The pipette part 120 may dispense the solution L into all the culture wells w-1 of the well plate P-1 even though the gap between the pipette tips T is wider than the gap between the culture wells w-1.

By the dispensing method, the liquid handling device 10 may enable efficient liquid handling of the dispenser unit 100. Various types of well plates may be applied to the liquid handling device 10, regardless of the size of the well plate P or the gap between the culture wells w.

Hereinafter, a dispensing process and an aspiration process of the liquid handling device 10 will be described in detail with reference back to FIGS. 1 to 9.

According to an embodiment, the liquid handling device 10 may actuate the dispenser unit 100 and the gripper unit 200 and perform liquid handling under the control of the controller 40.

<Dispensing process of Liquid handling device 10>

Before the liquid handling device 10 is actuated, the dispenser unit 100 and the gripper unit 200 may be stopped in respective standby positions.

In case that the liquid handling device 10 starts operating, the pipette part 120 may move down, to couple the pipette tips T loaded on the pipette tip loading part 130 to the pipette coupling portion 123.

In some embodiments, the gripper unit 200 may grip the well plate P loaded on the loading part 310 and move the well plate P from the loading part 310 to the worktable 320.

After arranging the well plate P on the worktable 320, the gripper unit 200 may grip only the plate cover C covering the well plate P and return to the standby position of the gripper unit 200.

The dispenser unit 100 may move to the reservoir unit 400, and the pipette part 120 may move down to take the solution L through the aspiration operation. For example, the solution L may be stored by a preset volume in the pipette tips T of the pipette part 120.

At this time, when the pump unit 500 is set to be used, the pump unit 500 may be activated and may supply the solution stored in the sterilization bag 600 to the reservoir unit 400 as the level of the solution L stored in the reservoir unit 400 is lowered.

In case that a mixing operation of the pipette part 120 is set to be performed at least once, the pipette part 120 may perform the aspiration operation after mixing the solution L in the reservoir unit 400 at least once.

After aspirating the solution L, the dispenser unit 100 may move to the worktable 320, and the pipette part 120 may move down to approach the well plate P arranged on the worktable 320. The pipette part 120 may dispense the solution L into the plurality of culture wells w through the expulsion operation and return to the standby position of the dispenser unit 100.

The gripper unit 200 may move from the standby position of the gripper unit 200 to the worktable 320, cover the well plate P with the plate cover C, grip the well plate P, and move to the unloading part 330.

By repeating those processes a number of times specified by a user, the task of dispensing the solution L into the plurality of well plates P may be continuously performed.

Upon completion of the dispensing process, the dispenser unit 100 may move to the waste disposal unit 700 and separate and discard the pipette tips T from the pipette coupling portion 123.

<Aspiration process of Liquid handling device 10>

Before the liquid handling device 10 is actuated, the dispenser unit 100 and the gripper unit 200 may be stopped in respective standby positions.

In case that the liquid handling device 10 starts operating, the pipette part 120 may move down, to couple the pipette tips T loaded on the pipette tip loading part 130 to the pipette coupling portion 123.

In some embodiments, the gripper unit 200 may grip the well plate P loaded on the loading part 310 and move the well plate P from the loading part 310 to the worktable 320. The well plate P loaded on the loading part 310 may have the solution L injected into the culture wells w.

After arranging the well plate P on the worktable 320, the gripper unit 200 may grip only the plate cover C covering the well plate P and return to the standby position of the gripper unit 200.

The dispenser unit 100 may move to the worktable 320, and the pipette part 120 may move down to approach the well plate P arranged on the worktable 320. The pipette part 120 may immerse the pipette tips T in the solution L in the culture wells w and then take the solution L through the aspiration operation.

At this time, in case that it is set to collect the solution L, the dispenser unit 100 may move to the empty reservoir unit 400, and the pipette part 120 may expel the solution L.

In another example, in case that it is set not to collect the solution L, the dispenser unit 100 may move to the waste disposal unit 700, and the pipette part 120 may expel and discard the solution L. At this time, the waste disposal unit 700 may activate a drain pump (not shown) to expel the discarded solution L out of equipment.

After the pipette part 120 expels the solution L, the dispenser unit 100 may return to the standby position of the dispenser unit 100.

The gripper unit 200 may move from the standby position of the gripper unit 200 to the worktable 320, cover the well plate P with the plate cover C, grip the well plate P, and move to the unloading part 330.

By repeating those processes a number of times specified by the user, the task of aspirating and removing the solution L from the plurality of well plates P may be continuously performed.

Upon completion of the aspiration process, the dispenser unit 100 may move to the waste disposal unit 700 and separate and discard the pipette tips T from the pipette coupling portion 123.

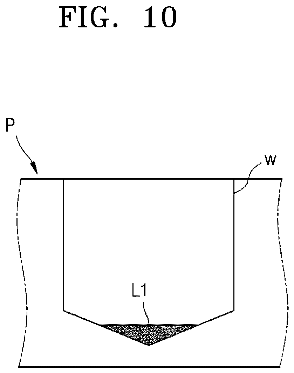

FIG. 10 is a view of a process of coating a well plate using the dispenser unit of FIG. 2.

The liquid handling device 10 may dispense a coating solution L1 into the culture well w of the well plate P according to the dispensing process.

The coating solution L1 may be applied to coat a surface of the culture well w.

According to an embodiment, the coating solution L1 may be a solution containing a protein. The protein may be a recombinant protein having a hydrophobic end and a hydrophilic end. The hydrophobic end of the protein may be bound to the surface of the culture well w. In this way, at least a portion of the surface of the culture well w may be coated.

A certain period of time may be required for the surface of the culture well w to be coated with the coating solution L1, and an environment with appropriate temperature and humidity may be required.

For example, after dispensing the coating solution L1 in a certain volume into the culture well w, the well plate P may be left to stand by at room temperature for 4 hours while being covered with the plate cover C, and the surface of the culture well w may be coated with the coating solution L1.

In some embodiments, the liquid handling device 10 may coat the culture well w by dispensing an excessive amount of coating solution L1 into the culture well w according to the dispensing process.

In this instance, a surface area in contact with the coating solution L1 inside the culture well w may increase, so that the surface of the culture well w may be coated in a wider area.

After coating the surface of the culture well w with the coating solution L1, the liquid handling device 10 may remove the coating solution L1 remaining in the culture well w according to the aspiration process.

Afterwards, the liquid handling device 10 may perform a washing process of washing the culture well w of the well plate P using a buffer.

The washing process may be performed in a manner that the dispenser unit 100 dispenses the buffer into the well plate P arranged on the worktable 320 and then aspirates the buffer again from the well plate P. The culture well w may be washed a plurality of times by repeating the process in which the dispenser unit 100 dispenses and aspirates the buffer.

By the washing process, the coating solution L1 remaining in the culture well w may be completely removed, and the surface of the culture well w may be completely coated. Hereinafter, the coating solution L1 bound to the surface of the culture well w may be referred to as a 'coating material.'

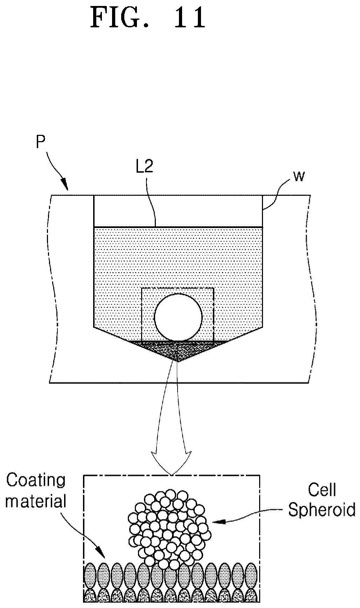

FIG. 11 is a view of a process of seeding and culturing cells using the dispenser unit of FIG. 2.

According to an embodiment, the coating solution L1 may contain a protein, and the protein may be a recombinant protein having a hydrophobic end and a hydrophilic end. Cells may bind to the hydrophilic end of the protein.

The liquid handling device 10 may dispense a culture medium L2 into the culture well w of the well plate P according to the dispensing process.

The culture medium L2 may be a medium mixed with cells. The cells may include one of muscle cells, blood cells, hepatocytes, nerve cells, skin cells, or alveolar cells. However, the cells are not limited to this, and may include more diverse types of cells.

To obtain cell clusters with a uniform size during a cell culture process, the cells need to be distributed and seeded in certain quantities into each culture well w.

Therefore, the liquid handling device 10 need to take the culture solution L2 by evenly mixing the cells into the culture medium L2.

The liquid handling device 10 may be set to perform a mixing operation of the pipette part 120, and the pipette part 120 may mix the culture medium L2 and the cells in the reservoir unit 400, aspirate the culture medium L2, and dispense the culture medium L2 into the culture well w.

The cells injected into the culture well w may bind to a coating material.

At this time, a binding force between the coating material and the cells may be weaker than a binding force between the cells, and thus, the cells may aggregate over time to form a spheroidal cell cluster.

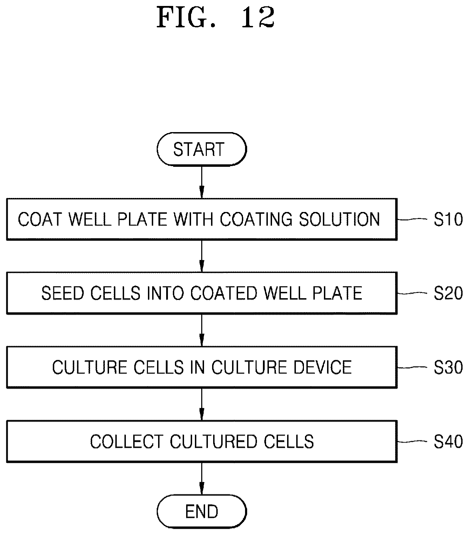

FIG. 12 is a flowchart of a cell culturing method using a liquid handling device according to an embodiment.

Hereinafter, a cell culture method using the liquid handling device 10 will be described with reference to FIGS. 1 to 12.

A cell culture method using the liquid handling device 10 may include coating a well plate with a coating solution (S10), seeding cells into the coated well plate (S20), culturing the cells seeded in the well plate in a culture device (S30), and collecting the cultured cells (S40).

In coating the well plate with the coating solution (S10), the liquid handling device 10 may coat the well plate P with the coating solution L1 under the control of the controller 40.

By virtue of the characteristics of the coating solution L1 described above in FIG. 11, the cells may be stably placed in the culture well w of the well plate P. The cells may easily aggregate into the spheroidal shape, forming a functionally superior cell spheroid.

In seeding the cells into the coated well plate (S20), the liquid handling device 10 may seed the cells into the plurality of culture wells w.

According to an embodiment, the liquid handling device 10 may seed the cells into the culture wells w of the well plate P by performing the dispensing process under the control of the controller 40.

The liquid handling device 10 may perform the dispensing process by operating the controller 40, and optionally, may be set to perform a mixing operation of the pipette part 120 to sufficiently mix the cells with the culture medium L2, aspirate the culture medium L2, and then dispense the culture medium L2 into the culture wells w of the well plate P. Accordingly, the number of cells seeded into the culture wells w may be maintained uniform.

In culturing the cells in the culture device (S30), the well plate P in which the cells have been seeded may be left in the culture device 20 for a certain period of time, so that the cells may be cultured.

As illustrated in FIG. 11, the cells seeded in the culture well w may be gradually separated from the coating material, and may bind to other surrounding cells, forming a cell cluster.

According to an embodiment, the cell cluster may be a cell spheroid.

The cell spheroid may form a structure similar to the shape of cells that constitute tissues in vivo, and may maintain the original function of the cells.

In case of in vivo injection of the cell spheroid, tissue engraftment rate and cell survival rate may be improved.

The cell spheroid may be densely packed cells. Therefore, the cell spheroid may have an advantage of being rich in extracellular matrix that mediates intercellular signaling and increasing the amount of effectors (cytokines, growth factors, and the like) produced in the cells.

In collecting the cultured cells (S40), the collection device 30 may collect the completely cultured cell clusters from the well plate P.

According to an embodiment, the collection device 30 may be configured as the liquid handling device 10. The liquid handling device 10 may collect the cell clusters by performing the aspiration process under the control of the controller 40.

The liquid handling device 10 may perform the aspiration process by operating the controller 40, and optionally, may be set to collect the solution L, so as to aspirate the fully cultured culture medium L2, and expel the culture medium L2 into the reservoir unit 400. Accordingly, the cell spheroids contained in the culture medium L2 may be collected.

In some embodiments, the quality of the cell spheroids may be determined by reproducibility of shape and size of the cell spheroids. The cell spheroids must be uniform in size and form a complete spheroidal shape each time the cell spheroids are manufactured.

The size of the cell spheroid may be proportional to the size of the culture well w and the number of cells seeded. The shape of the cell spheroid need to have a constant number of cells packed, and an environment favorable for the cells to aggregate into the spheroidal shape may be required.

A liquid handling device according to an embodiment may improve quality of cell spheroids by precisely performing liquid handling of a coating solution L1 and a culture medium L2.

A liquid handling device according to an embodiment may increase workers' convenience by continuously processing some of steps of a cell culture method through automation of liquid handling. The production speed and yield of the cell spheroids may be improved to enable mass production.

As such, the disclosure has been described with reference to an embodiment shown in the drawings, but is merely illustrative, and it will be understood by those of skilled in the art that various modifications and variations of the embodiment may be made. Therefore, the true scope of the disclosure should be determined only by the appended claims.

## Claims

1. A liquid handling device comprising:
a well plate having a plurality of culture wells;
a work unit configured to move the well plate along a preset path;
a dispenser unit configured to dispense or aspirate at least one of a coating solution and a culture medium into or from the well plate using pipette tips;
a gripper unit configured to grip the well plate and move the well plate along a preset path; and
a reservoir unit storing the at least one of the coating solution and the culture medium and supplying the same to the dispenser unit.

2. The liquid handling device of claim 1, wherein
the dispenser unit
dispenses the coating solution stored in the reservoir unit into the well plate, and dispenses the culture medium into the well plate coated with the coating solution.

3. The liquid handling device of claim 2, wherein
the dispenser unit
dispenses the culture medium after aspirating the dispensed coating solution.

4. The liquid handling device of claim 2, wherein
the dispenser unit
mixes the culture medium stored in the reservoir unit with cells, and then, dispenses the culture medium mixed with the cells into the well plate.

5. The liquid handling device of claim 2, wherein
the well plate has a coating surface which is coated with the coating solution in each of the culture wells, with the cells in the culture media aggregating into a spheroidal shape on the coating surface.

6. The liquid handling device of claim 1, wherein
the work unit comprises:
a loading part on which the well plate is loaded;
a worktable on which the well plate is placed, such that the coating solution or the culture medium is dispensed into the well plate; and an unloading part to which the dispensed well plate is unloaded.
